# FASCICULE DE BREVET EUROPEEN

(11) **EP 2 237 731 B1**
(45) Date de publication et mention de la délivrance du brevet: **24.02.2016**
(21) Numéro de dépôt: 08856891.0
(22) Date de dépôt: 03.12.2008
(51) Int. Cl.: A61B 18/20

(54) **DISPOSITIF DE TRAITEMENT THERMIQUE DERMATOLOGIQUE PAR FAISCEAU LASER**
VORRICHTUNG ZUR DERMATOLOGISCHEN WÄRMEBEHANDLUNG MIT EINEM LASERSTRAHL
DEVICE FOR DERMATOLOGICAL THERMAL TREATMENT USING A LASER BEAM

(30) Priorité: 03.12.2007 FR 0759521
(43) Date de publication de la demande: 13.10.2010
(73) Titulaire: Vivatech, 75008 Paris (FR)
(72) Inventeur: CORNIL, Alain, F-13090 Aix En Provence (FR); GOSSE, Alban, F-13105 Mimet (FR); PERONNE, Patrick, F-75014 Paris (FR)
(74) Mandataire: Barbot, Willy
(86) Numéro de dépôt international: PCT/EP2008/066729
(87) Numéro de publication internationale: WO 2009/071592

(56) Documents cités:
- EP-A- 1 354 573
- EP-A- 1 744 349
- EP-A- 1 748 471
- WO-A-00/53114
- WO-A-98/51235
- US-A1- 2002 138 119
- US-A1- 2007 179 571

## Description

La présente invention concerne le domaine du traitement de plaies de la peau d'un patient par faisceau laser et, plus spécifiquement un dispositif pour un tel traitement.

Il est connu, dans l'état de la technique, différentes solutions de cicatrisation par apport d'une énergie externe. Elles consistent à appliquer une énergie lumineuse émise par une source laser sur les tissus de façon à chauffer de manière sélective des parties précises de la peau, et ce, avec des temps de tir très réduits (inférieurs à la seconde). Le principe de sélectivité permet ainsi de cantonner l'élévation de température aux zones du tissu irradiées.

Les faisceaux laser ont une longueur d'onde choisie en fonction de la région à traiter. Dans le cas de l'épilation laser, la longueur d'onde correspond aux pics d'absorption de la mélanine, constituant principal du bulbe pileux.

Le laser agit en produisant une impulsion de lumière laser captée sélectivement par la lésion à traiter en fonction de sa couleur et sans causer de dommage aux tissus environnants.

On connait également de la demande de brevet européen EP265470 un dispositif pour l'assemblage des lèvres d'une plaie comportant un laser dont la longueur d'onde d'émission est choisie de manière à pouvoir réaliser une soudure tissulaire et assembler les lèvres d'une plaie, et une pièce de maintien apte à être assujettie au tissu autour de la plaie de manière à maintenir affrontées les lèvres de cette dernière, au moins pendant l'exposition de la plaie audit rayonnement laser.

L'idée maîtresse est de souder la peau comme les vaisseaux, en utilisant une énergie laser suffisante pour obtenir une élévation de température tissulaire au delà de 60°C, apte à obtenir la dénaturation et l'inter-digitation des fibres de collagène. De nos jours, cette température est reconnue comme étant le niveau au-delà duquel on obtient un dommage thermique irréversible pour des durées de chauffage supérieures à la seconde, entraînant la coagulation tissulaire et la nécrose par brûlure qui ralentissent la cicatrisation et grèvent sa qualité.

De manière générale, l'état de la technique vise à appliquer une énergie électromagnétique émise par une source laser ou RF (fréquence radio) sur les tissus de façon à chauffer de manière sélective des parties précises de la peau avec plusieurs approches différentes :
- Pour les techniques visant de petites structures, les temps d'irradiation sont très réduits, inférieurs au temps de relaxation thermique de la zone ciblée. C'est le cas des systèmes d'épilation et de traitement des lésions vasculaires. Dans ces deux cas, les faisceaux laser ont une longueur d'onde choisie en fonction de la région à traiter. Dans le cas de l'épilation laser, la longueur d'onde correspond aux pics d'absorption de la mélanine, constituant principal du bulbe pileux (documents US 5595568 et 5735844). Le laser vasculaire a lui une longueur d'onde choisie pour sa plus grande absorption par l'hémoglobine.
- Pour les structures en profondeur (derme par exemple) : Le principe de sélectivité est obtenu par association de la source RF ou laser avec un système de refroidissement superficiel assurant un gradient inversé de température et préservant les structures en surface. C'est le cas des techniques de remodelage RF (document US 5755753) ou laser.
- Une troisième approche pour la sélectivité combine l'une et l'autre des solutions et peut aussi associer l'application d'une pression afin de réduire la quantité de sang, et donc d'hémoglobine pouvant interférer avec le traitement, dans la peau (document US 5630811).

En conclusion, dans l'état de la technique, le faisceau laser, présentant généralement un profil gaussien classique, a un inconvénient majeur. En effet, la répartition de l'énergie s'apparente à une fonction gaussienne, ce qui a pour effet direct de chauffer la surface de la peau de manière non uniforme et sélective avec un pic de température important au centre de la zone irradiée et donc d'avoir un gradient de température trop important par rapport à la précision de la plage de température dans laquelle on désire se placer.

Le document US2007/179571, qui est considéré comme l'état de l'art le plus proche, décrit un dispositif de traitement dermatologique selon le préambule de la revendication 1.

Un but de la présente invention est de chercher, à améliorer l'état de la technique afin de résoudre le problème cité précédemment. L'invention est comme définie dans le jeu de revendications de la page 35.

A cet effet, il est prévu, selon l'invention, un dispositif de traitement dermatologique comprenant une source d'énergie, la source d'énergie délivrant un faisceau laser quasi-gaussien, caractérisé en ce qu'il comprend des moyens de mise en forme du faisceau entre la source d'énergie et une source à traiter, les moyens de mise en forme étant adapté pour convertir le faisceau en un faisceau dont la puissance est répartie de manière homogène sur une surface donnée, et en ce que le dispositif comprend en outre un bloc optique détachable et interchangeable.

Cette répartition homogène de la puissance permet d'obtenir un chauffage sélectif mais global et homogène sur la zone de tissu à traiter pour une activation de la cicatrisation du tissu.

Avantageusement, mais facultativement, l'invention comporte au moins l'une des caractéristiques suivantes :
- les moyens de mise en forme du faisceau laser sont adaptés pour convertir le faisceau en un faisceau de profil quasi-linéaire,
- les moyens de mise en forme du faisceau laser comprennent au moins un réseau de lentilles et une lentille cylindrique,
- le dispositif comprend une fibre optique placée devant la source d'énergie afin de diminuer la divergence de l'axe rapide de la source d'énergie,
- le dispositif comprend un moyen de pyrométrie,
- le dispositif comprend une interface d'utilisation comprenant au moins l'un des éléments suivants : un écran LCD, un bouton d'arrêt d'urgence, un bouton marche/arrêt, un système double/bouton, un voyant lumineux et un buzzer,
- la source d'énergie est une diode de longueur d'onde comprise entre 800 et 1800nm, et préférentiellement entre 810nm et 910nm,
- le dispositif comprend un moyen de batterie afin de rendre le dispositif autonome,
- le dispositif comprend un moyen de lecture RFID (Radio Fréquence IDentification) afin de sécuriser l'utilisation du dispositif,
- la portée du lecteur RFID inférieure à 5 mm,
- le dispositif comprend un microcontrôleur destiné à la gestion de la source d'énergie,
- le dispositif comprend un décroché en aval de la sortie des moyens de mise en forme du faisceau laser afin de garantir une distance entre la sortie des moyens de mise en forme et la zone à traiter,

L'invention concerne également un ensemble de traitement comprenant un dispositif selon l'invention et comprenant en outre un manchon de stérilisation.

L'invention concerne également un manchon de stérilisation.

L'invention concerne également un équipement de traitement dermatologique et un procédé de traitement dermatologique, tel que :
- l'équipement de traitement dermatologique comprend un dispositif selon l'invention et un support RFID, le support RFID étant destiné à transmettre au dispositif des informations concernant la zone à traiter,
- le dispositif comprend un microcontrôleur destiné à la gestion de la lecture du support RFID,
- le support RFID interagit avec la source d'énergie grâce à un contact,
- les paramètres de fonctionnement de la source d'énergie sont mémorisés dans le support RFID et communiqués au dispositif pour la commande de la source d'énergie,
- le support RFID comprend des moyens de stockage d'une information caractérisant un état d'une zone à traiter, l'information correspondant à l'un des états suivants :
   - la zone à traiter n'a pas été traitée,
   - la zone à traiter est en cours de traitement,
   - le traitement de la zone à traiter est terminé.
- le support RFID fonctionne dans la bande comprise entre 13 et 14 MHz,
- le dispositif, recevant l'information correspondant à l'état de la zone traiter, comporte des moyens d'empêcher une seconde utilisation du support adhésif muni d'un support RFID lorsque celui-ci a déjà été utilisé une première fois pour traiter la zone à traiter,
- le support RFID (92) est compris dans un support adhésif (90), le support adhésif (90) étant positionné à proximité d'une zone à traiter,
- le support RFID comprend des moyens empêchant une seconde utilisation du support adhésif si celui-ci a déjà été utilisé pour le traitement d'une zone à traiter,
- le support RFID (92) étant compris dans le manchon de stérilisation,
- le support RFID (92) étant compris dans un élément portatif,
- l'élément portatif est choisi parmi le groupe suivant : badge, téléphone, bipper, carte d'identification.

Il est également décrit un procédé de traitement dermatologique, tel que :
- le procédé est adapté de préférence pour une activation biochimique de la cicatrisation de plaies de la peau d'un patient, à l'aide d'un dispositif de traitement selon l'invention, le procédé comprenant l'étape d'application d'un faisceau laser sur une zone cutanée dudit patient, la puissance dudit faisceau étant de répartition homogène sur toute sa section,
- le procédé comprend en outre préalablement l'étape suivante : mettre le dispositif de traitement dans un manchon de stérilisation,
- le procédé comprend en outre les étapes suivantes :
   - Appliquer un support adhésif à proximité de la zone à traiter,
   - Choisir un bloc optique pour le dispositif,
   - Approcher le dispositif de la plaie pour que le dispositif de lecture RFID puisse lire les informations du support RFID,
   - Appliquer le faisceau laser sur la zone à traiter.

D'autres caractéristiques, buts et avantages de la présente invention apparaîtront à la lecture de la description détaillée qui va suivre, au regard des dessins annexés, donnés à titre d'exemples non limitatifs et sur lesquels:
- La figure 1 est un graphe présentant une relation logarithmique entre un temps d'exposition et une température pour obtenir l'induction d'une "Heat Shock Response",
- La figure 2 est un graphe présentant les différentes phases de cicatrisation d'une plaie cutanées,
- La figure 3 est un graphe présentant différents coefficients d'absorption des principaux chromophores en fonction des longueurs d'onde d'une source laser émise,
- La figure 4 est un graphe présentant la profondeur de pénétration (en pourcentage) d'un faisceau laser dans la peau en fonction de sa longueur d'onde,
- La figure 5 est un graphe présentant un profil de faisceau laser classique et un profil de faisceau laser selon l'invention,
- La figure 6 représente de manière schématique un dispositif de transformation d'un faisceau laser selon l'invention,
- Les figures 7 et 8 représentent des schémas représentatifs d'un dispositif de traitement dermatologique selon l'invention,
- La figure 9 est un est un schéma simplifié d'une bandelette de sécurité selon l'invention,
- La figure 10 est un exemple de dessin imprimable sur une bandelette de sécurité selon l'invention,
- La figure 11 est un diagramme schématique de la communication entre un support RFID et les deux microcontrôleurs d'un dispositif selon l'invention,
- Les figures 12a à 12c sont des graphes représentant différents cas de montée de température à la surface d'une zone de tissu traitée,
- Les figures 13 et 14 sont des graphes fonctionnels décisionnels des microcontrôleurs d'un dispositif selon l'invention.
- La figure 15 représente un dispositif et un manchon selon une réalisation particulière de l'invention,
- La figure 16 représente un dispositif et des éléments portatifs selon une réalisation particulière de la présente invention.

L'invention repose sur l'échauffement modéré afin d'effectuer le conditionnement thermique d'un volume cutané limité entourant et incluant une lésion cutanée présente lors du traitement ou à venir (dans le cas des incisions chirurgicales par exemple). Comme précisé précédemment, il ne s'agit pas de chauffer de façon sélective un ou plusieurs constituants de la peau, mais effectivement de chauffer dans sa globalité un volume situé sur l'épaisseur de la peau (épiderme, derme, couche supérieure de l'hypoderme) afin de générer une réponse biologique de l'ensemble du tissu au stress thermique ou conditionnement thermique. Le choix du faisceau et de tous les paramètres liés repose donc sur ces spécifications.

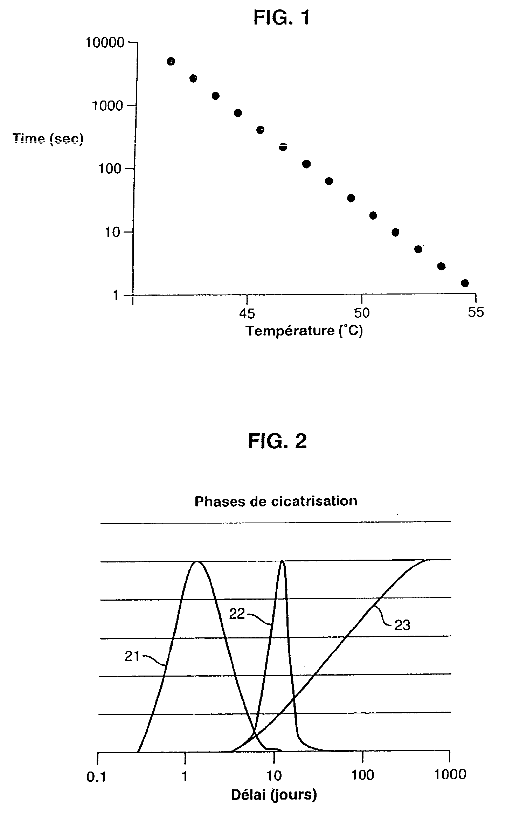

En référence à la figure 1, représentant une relation logarithmique entre un temps d'exposition en ordonnées (unités en seconde) et une température pour obtenir l'induction d'une réponse de choc thermique ("Heat Shock Response" en abscisse ; unités en degrés Celsius). La réponse de choc thermique est un mécanisme cellulaire permettant de maintenir une stabilité lors d'un choc par exemple thermique ; une réponse de choc thermique implique souvent la production de protéines de choc thermique (« Heat Sock Proteins », HSP), groupe de protéines pouvant servir à accélérer le processus de cicatrisation.

L'invention ne vise pas à modifier la structure cellulaire, mais à modifier le processus de cicatrisation en induisant la production de HSP. En effet, une hyperthermie non contrôlée peut aboutir rapidement à un dommage tissulaire et par conséquent à la dénaturation ou à la destruction du tissus. En se plaçant dans le cas d'un conditionnement thermique, il est prévu, selon l'invention, de produire une fièvre localisée dont la température maximale est contrôlée pour éviter l'apparition de dommages tissulaires.

En dirigeant une source d'énergie électromagnétique, comme un laser, vers la zone de tissu lésé, un conditionnement thermique qui va modifier le processus inflammatoire est induit. Le lien entre l'élévation contrôlée de température (hyperthermie modérée), l'expression des HSP et le processus d'inflammation a été déjà établi dans l'état de l'art.

En référence à la figure 2, le processus de cicatrisation se décompose en trois phases suivant la thrombose initiale :
- inflammation 21,
- prolifération 22, et
- remodelage 23.

En abscisse de ce graphe, est mis le temps écoulé depuis le début de la cicatrisation et en ordonnées un niveau de réponse à un choc thermique donné et donc un niveau de production de HSP.

Un procédé de traitement de plaies de la peau d'un patient par faisceau laser consiste à diriger une énergie lumineuse sur le tissu dans le but de générer une hyperthermie modérée et contrôlée le plus tôt possible dans le processus de cicatrisation afin de prévenir l'apparition de la rançon cicatricielle et d'accélérer la régénération tissulaire. Ceci plutôt que de corriger cette rançon tissulaire en intervenant une fois que la cicatrice est apparue. Le moment du traitement doit donc être choisi de telle façon à ce que l'effet soit optimum par rapport à la phase d'inflammation.

Il convient de définir une fenêtre de traitement optimale qui fait coïncider le pic d'expression des HSP avec la phase d'inflammation. Le pic d'expression des HSP pouvant intervenir jusqu'à vingt quatre heures suivant l'application du stress thermique, le conditionnement pourra s'appliquer jusqu'à vingt quatre heures avant que n'apparaisse la lésion.

### Choix du faisceau :

Le choix du faisceau et de tous les paramètres liés repose donc sur des spécifications précises. Un modèle de simulation numérique par méthode des éléments finis intégrant les interactions de la lumière avec les principaux chromophores localisés dans la région à traiter, a été réalisé afin de confirmer les choix théoriques.

### Choix de la longueur d'onde :

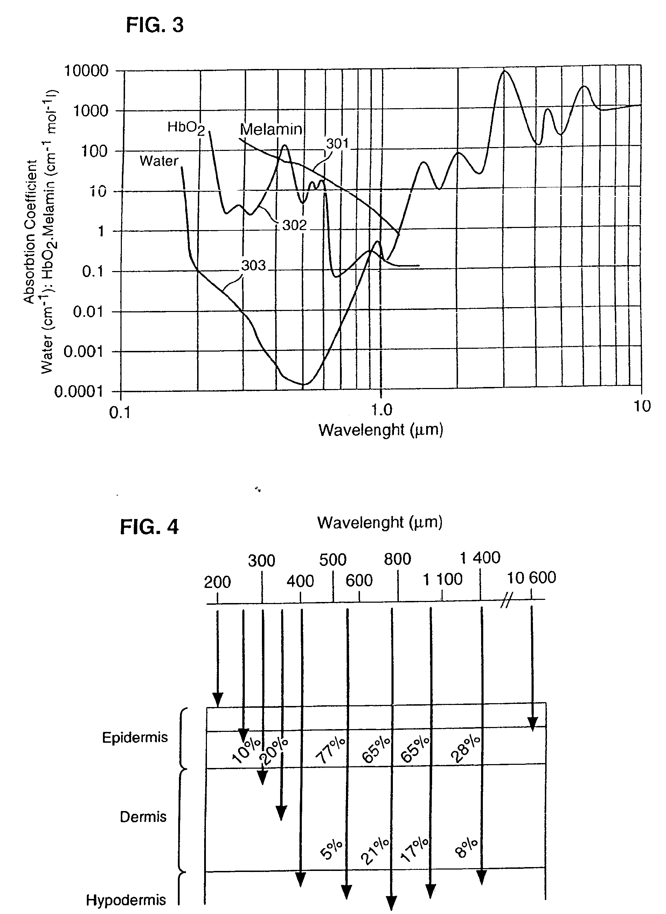

La figure 3 est un graphe avec trois courbes représentant, en fonction de la longueur d'onde de l'énergie apportée (en abscisse), le coefficient d'absorption de cette énergie (en ordonnées), et ceci pour les éléments suivants :
- la mélanine, représentée par la courbe 301,
- l'hémoglobine représentée par la courbe 302,
- l'eau représentée par la courbe 303.

La figure 4, quant à elle, est un graphe présentant pour chaque longueur d'onde le taux d'absorption de chaque couche cutanée.

En référence à ces figures, l'absorption de la lumière par les différents chromophores varie donc considérablement en fonction de la longueur d'onde.

Les mélanocytes étant situés dans la couche basale de l'épiderme, la longueur d'onde doit être supérieure à 800nm si l'on veut pouvoir traverser cette couche sans être absorbé totalement. Pour les longueurs d'onde inférieures à 590nm, l'hémoglobine est le chromophore prédominant et présente donc une forte absorption. Par contre, dans le rouge et le proche infrarouge (longueur d'onde entre 600nm et 1000nm), l'absorption est relativement faible, car ni le sang, ni l'eau n'absorbent dans ce domaine de longueur d'onde. Enfin, pour les longueurs d'onde supérieures à 1800nm, l'absorption de l'eau est extrêmement importante et cette absorption devient le facteur prédominant.

La plage de longueur d'onde doit donc se situer entre 800nm et 1800nm. Dans un mode privilégié la longueur d'onde sera égale à 810nm ou 910nm. Avantageusement, une longueur d'onde de 1200nm permettrait de minimiser l'absorption par la mélanine, mais la technologie actuelle ne permet pas de fournir un faisceau de cette longueur d'onde dont la source est une diode laser avec une puissance suffisante.

### Choix de la géométrie du spot :

La géométrie du spot laser doit être également étudiée de façon à pouvoir chauffer en profondeur afin de stimuler l'ensemble des tissus impliqués dans le processus de cicatrisation cutanée : hypoderme, derme et épiderme. La profondeur étant quasi proportionnelle au diamètre du spot laser, on privilégiera donc un diamètre (dans le cas de spot rond) supérieur ou égal à 3mm. Idéalement, la géométrie du spot peut également être adaptée à la géométrie de l'endroit à traiter et permettre une répartition la plus homogène possible de l'énergie lumineuse dans le tissu ciblé. Dans un mode de réalisation privilégié, on choisira une géométrie de spot rectangulaire de largeur au moins égale à 3mm et de longueur pouvant atteindre plusieurs centimètres, permettant ainsi le traitement de cicatrices linéaires.

### Précision de la température du volume chauffé et profil du faisceau laser :

L'invention réside également dans la précision de température atteinte dans le volume chauffé. Le modèle numérique développé permet d'optimiser l'ensemble des paramètres pour atteindre une température minimale de 45°C (température nécessaire pour provoquer le stress thermique) et maximale de 55°C. Comme décrit dans l'état de l'art, dépasser 60°C peut provoquer une dénaturation protéinique et aura un effet contraire à l'effet escompté. La température maximale de 55°C permet donc de rester au dessous de cette valeur seuil.

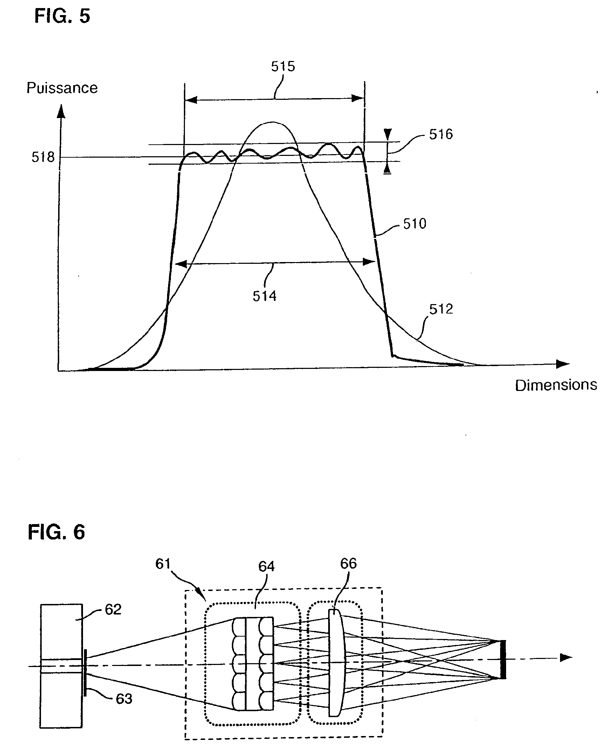

Pour ces raisons, et en référence à la figure 5, présentant un profil gaussien classique de faisceau (courbe 512) et un profil de faisceau d'un dispositif selon un mode de réalisation possible de l'invention (courbe 510), un profil de faisceau gaussien classique n'est pas le plus adapté. En effet, comme son nom l'indique, la répartition de l'énergie s'apparente à une fonction gaussienne, ce qui a pour effet direct de chauffer la surface de la peau de manière non uniforme avec un pic de température important au centre de la zone irradiée et donc d'avoir un gradient de température trop important par rapport à la précision de la plage de température dans laquelle on désire se placer.

Par voie de conséquence, on privilégiera donc un profil quasi-linéaire (« top-hat » ou « flat-top» en anglais) 510 plutôt que gaussien de façon à homogénéiser la température obtenue à la surface de la peau, et donc dans le volume chauffé.

Ce profil de puissance quasi-linéaire, représenté par la courbe 510, permet d'avoir un chauffage plus homogène sur la zone de chauffe plutôt qu'un chauffage sélectif. Ce profil est défini par deux éléments :
- un rapport d'une largeur de front 515 sur une largeur à mi hauteur (FWHM = Full Width Half Maximum) 514,
- un taux de variation 516, le long de la largeur de front 515, et
- une puissance nominale 518, qui est la puissance moyenne le long de la largeur de front 515.

Une caractéristique d'un profil quasi-linéaire est d'avoir un taux de variation 516 minimum le long de la largeur de front, tout en gardant un rapport entre largeur de front/largeur à mi-hauteur maximal. Avantageusement un profil quasi-linéaire a un taux de variation inférieur à 5% pour un rapport largeur de front/largeur à mi-hauteur supérieur à 90%.

La géométrie du spot dépend de l'indication d'utilisation. Dans un mode de réalisation privilégié et dans une configuration choisie de traitement d'incision, les dimensions du spot sont de 20mm sur 4mm, et le taux de variation inférieur à +/-20% de la puissance nominale 518.

Afin d'obtenir ce profil particulier, il est prévu selon l'invention, un moyen de mise en forme du faisceau laser émanant de la diode (le faisceau laser ayant un profil gaussien en sortie de diode). Un tel moyen est représenté sur la figure 6.

Ainsi le moyen de mise en forme du faisceau laser 61 selon un mode de réalisation possible de l'invention est placé en aval d'une diode laser 62 caractérisée avantageusement par une largeur d'émission de 200 µm sur 5 µm et une divergence de 8x35°. Une fibre optique 63 placée devant la diode sert de lentille cylindrique afin de diminuer la divergence de l'axe rapide de la diode à avantageusement environ 5°. Le faisceau laser passe ensuite dans le moyen de mise en forme du faisceau laser 61 comprenant un système de deux réseaux de lentilles 64 et d'une lentille cylindrique 66 ; ce qui permet de diviser le faisceau d'origine en autant de faisceau qu'il y a de lentilles dans le réseau de lentilles 64. Chaque sous-faisceau est focalisé à la distance souhaitée et la superposition de tous ces faisceaux permet d'obtenir un profil quasi-linéaire.

La distance à laquelle est focalisé le faisceau dépend principalement de la focale de la lentille cylindrique. Mais l'homogénéité du profil dépend, en plus de cette focale, des focales des deux réseaux de lentilles. D'autre part pour obtenir un profil de faisceau quasi-linéaire le plus homogène possible il faut que le faisceau d'entrée illumine le maximum de lentilles du réseau (plus le faisceau est divisé en sous-faisceaux, plus la somme de ces sous-faisceaux tendra vers un profil quasi-linéaire). Ainsi, il existe un compromis entre la distance entre la diode et le premier réseau de lentilles et l'angle de divergence de façon à maximiser l'homogénéité du profil quasi-linéaire tout en réduisant la longueur du système final.

### Réalisation privilégiées :

Afin de rendre la technique accessible au plus grand nombre d'utilisateurs, la conception du dispositif prend en compte sa facilité d'utilisation et sa sécurité d'emploi en toutes circonstances, et dans l'ensemble des lieux dans lequel il pourrait être utilisé, du simple cabinet de consultation jusqu'à l'environnement stérile d'un bloc opératoire. Les utilisateurs du dispositif pouvant être amenés à voyager ou se déplacer régulièrement, le transport du dispositif en termes d'encombrement est également primordial. De plus, comme décrit précédemment, il est prévu selon l'invention d'appliquer une source lumineuse le plus tôt possible dans le processus de cicatrisation afin de prévenir l'apparition de la rançon cicatricielle, plutôt que de corriger en intervenant une fois que la cicatrice est apparue. Pour cela, il est nécessaire de fournir à l'utilisateur un dispositif médical lui permettant d'intervenir le plus tôt possible, c'est-à-dire directement au bloc opératoire, en environnement stérile.

Il convient donc de concevoir un dispositif médical d'encombrement réduit, portable et utilisable avec une seule main, autonome (sans fil) et ne remettant pas en cause la propreté ou la stérilité du milieu dans lequel il est utilisé.

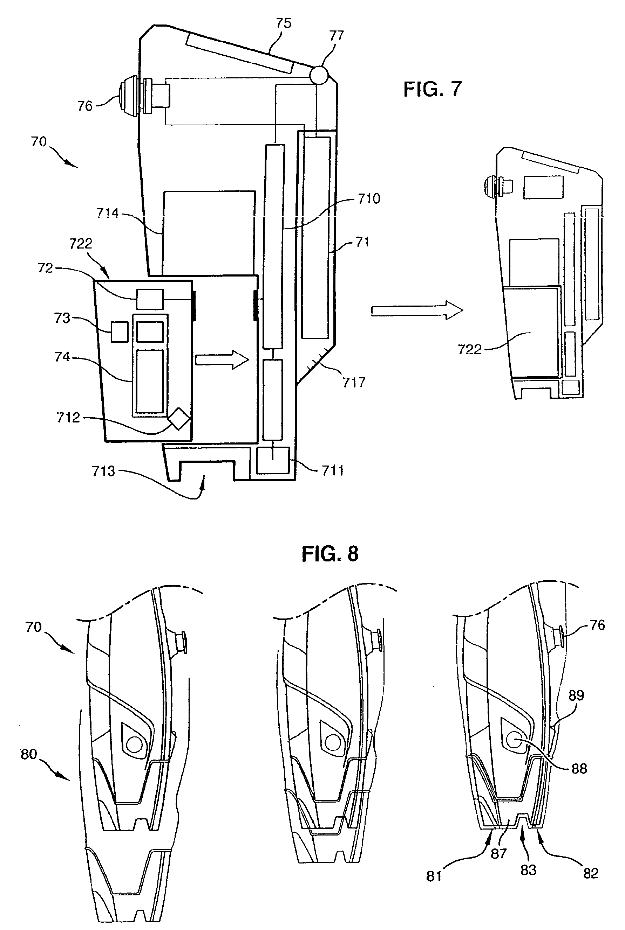

En référence aux figures 7 et 8, nous allons décrire une réalisation d'un dispositif selon l'invention.

Ainsi, un dispositif 70 de traitement dermatologique selon l'invention comprend un bloc optique 722, fermé, comprenant au moins un système laser 72 composé d'une diode laser de puissance de longueur d'onde comprise entre 750nm et 1400nm et de puissance supérieure à 1 W et inférieure à 25 W, et un moyen de transformation 74 comprenant un réseau de lentille ou un réseau de phase. Le système laser 72 et le moyen de transformation 74 sont destinés à émettre un faisceau laser présentant les caractéristiques avantageuses précédemment citées. Le bloc optique 722 peut également comprendre un laser de visée 73, par exemple de puissance inférieure à 1 mW afin que ce laser de visée n'ait qu'un rôle de confort et qu'il ne génère aucune réponse biologique. Afin de rendre ce laser de visée 73 visible, sa longueur peut être choisie par exemple entre 480nm et 650nm (couleur rouge).

Il est prévu, selon l'invention que le bloc optique 722 soit amovible et interchangeable afin de faciliter la maintenance du dispositif de traitement 70 et de garantir sa flexibilité (possibilité de changer de type de diode selon le traitement souhaité). Ainsi, il est prévu selon l'invention de fournir à un utilisateur du dispositif (par exemple un praticien) un panel de blocs optiques ayant des configurations différentes tout en gardant les contraintes liées à la sécurité (aucun réglage possible par l'utilisateur lui-même).

Le dispositif de traitement 70 comprend également un moyen de batterie 71 permettant une entière autonomie énergétique du dispositif. Ce moyen de batterie peut être interchangeable.

Le dispositif de traitement 70 comprend un ou plusieurs circuits électroniques 710 ayant pour fonction la gestion de l'alimentation générale du dispositif et celle des différents composants compris dans le dispositif.

Le dispositif de traitement comprend un lecteur RFID 711 (sous forme d'antenne RFID par exemple) permettant de sécuriser l'ensemble du dispositif en ne permettant le tir qu'à proximité d'un support (ou « tag ») RFID apposé à proximité de l'endroit à traiter, évitant ainsi tout risque de dommage pour l'utilisateur, le patient et l'entourage ; et de sécuriser le traitement en lisant les paramètres préenregistrés dans le support, ne permettant pas au praticien de modifier accidentellement tout paramètre pouvant être dangereux pour le patient (puissance et durée du tir principalement). Les paramètres de tir du laser sont commandés en fonction de la nature des informations transmises par le support RFID, qui contient des paramètres permettant d'adapter la puissance, la durée et le nombre des tirs en fonction des choix de l'utilisateur.

L'utilisateur sélectionnera le support en fonction de la nature du traitement à effectuer, et le tag sélectionné délivre au récepteur un signal ou une information qui pilotera les tirs du laser. Le support est constitué par exemple par un timbre (ou, en anglais « patch ») formé par un morceau de tissu adhésif comprenant une étiquette radiofréquence (en anglais « RFID ») constituée par un dessin d'antenne permettant un couplage inductif pour l'alimentation d'un composant haute fréquence, et la transmission du signal haute fréquence émis par ce composant.

Le support doit être positionné à proximité de la zone à traiter de manière à ce que le dispositif de traitement 70 soit à portée d'interaction avec l'étiquette pendant la durée du traitement.

Ces supports RFID sont largement décrits dans l'état de l'art. Cependant, l'invention prévoit que le support RFID ait également pour fonction d'automatiser le procédé. En fait, le support RFID possède un identifiant qui renvoie vers une table de paramétrage localisée dans le soft du laser dans laquelle sont prédéfinies la puissance et la durée de tir, mais également le type de patient qui doit être traité (pour une vérification de sécurité de la part du praticien). Lors de l'utilisation du dispositif, l'utilisateur n'a qu'à choisir la bandelette de sécurité (qui contient les supports RFID) en fonction du phototype du patient et de l'indication (cicatrice, traitement de l'acné, remodling cutané, etc.). Contrairement aux autres lasers disponibles sur le marché, aucun ajustage des paramètres par l'utilisateur n'est rendu possible. Le traitement ne repose ainsi que sur le choix de la bandelette qui contient l'identifiant du traitement.

Avantageusement, la distance entre le support RFID et le dispositif de traitement 70 est comprise entre un et quinze centimètres. L'évaluation de la distance est réalisée par une portée limitée des moyens d'interaction entre le support et le dispositif de traitement 70, ou par un télémètre, par exemple un télémètre à ultrasons, intégré dans le dispositif.

Le dispositif de traitement comprend également un pyromètre 712 permettant de contrôler l'élévation de température pouvant provoquer une brûlure superficielle (température supérieure à 60°C). Le pyromètre 712 permet de mettre en place une boucle fermée sur le faisceau laser. En effet, Le pyromètre 712 est un organe de sécurité qui « surveille » la température à la surface de la peau. Il intervient afin de « bloquer » ou désactiver le tir si la température atteint une valeur seuil prédéfinie. Le pyromètre 712 peut être également utilisé de manière plus fine dans une boucle de régulation fermée afin de réajustement des paramètres laser.

De manière générale, les températures obtenues en surface et en profondeur de l'épiderme vont dépendre de plusieurs paramètres, tels que :
1 - la longueur d'onde de laquelle dépendent l'absorption et la diffusion de la lumière par les différents chromophores (eau, hémoglobine, mélanine),
2 - la puissance du laser qui permet de chauffer plus ou moins en profondeur,
3 - la géométrie et les dimensions du spot, qui influent sur la répartition de la chaleur en profondeur,
4 - le profil du faisceau qui a un impact direct sur le gradient de température obtenu sur une section de faisceau (profil gaussien, profil quasi-linéaire),
5 - le temps de tir qui, associé, à une puissance donnée, favorise s'il est long un système de chauffage par diffusion thermique,
6 - enfin, les paramètres directement associés au patient, le plus important étant a priori son phototype.

Il ne peut donc pas y avoir de corrélation générale entre la température à la surface de l'épiderme et de celle des couches inférieures. Par contre, si l'on fige tous les paramètres associés au laser (points 1 à 5), il est possible de « prédire » l'élévation de température dans les tissus à partir d'un facteur humain et donc du phototype.

Il est prévu de surcroît, une fenêtre optique transparente 713 dans la gamme de longueur d'onde comprise entre 480 nm et 1,4 µm. Dans un mode privilégié, le bromide de potassium transparent pour des longueurs d'ondes allant de 450nm à 10µm pourra être choisi.

Un système de mise en veille du dispositif peut également être rajouté, dès lors que l'appareil n'est pas utilisé pendant un temps prédéterminé. Le mode de mise en veille est interrompu, dès lors que l'utilisateur appuie sur l'un des deux boutons de tir. Ce type de système permet d'une part d'économiser la batterie, mais surtout d'éviter que le laser de visée (même de faible puissance) ne puisse provoquer des dommages oculaires.

Le dispositif de traitement peut en outre comprendre un système de refroidissement composé d'un radiateur 714 interne relié directement ou via un caloduc à la diode laser. En outre, il est prévu selon une réalisation possible de l'invention un dispositif de ventilation naturelle et/ou forcée pour évacuer la chaleur émise par la diode laser hors du dispositif et de façon stérile, afin de ne pas compromettre la stérilité du champ opératoire au dessus duquel est utilisé le dispositif de traitement 70.

Avantageusement, il est prévu d'équiper le dispositif d'une IHM (Interface Homme-Machine) afin de pouvoir renseigner l'utilisateur sur les réglages d'utilisation (longueur d'onde, paramètre contenu sur un support RFID capté par le dispositif de traitement RFID, température mesurée avec le pyromètre).

Nous allons détailler une IHM d'un dispositif de traitement selon l'invention. Avantageusement l'IHM d'un dispositif de traitement selon l'invention comprend les éléments suivants :
- un écran LCD 75, qui permet un retour d'information à l'utilisateur sur les réglages et paramétrages d'utilisation du dispositif selon l'invention (l'écran LCD peut être tactile),
- un bouton d'arrêt d'urgence 76, permettant à l'utilisateur un arrêt rapide du dispositif en cas d'urgence,
- un bouton marche/arrêt 77 pour la mise en marche ou l'arrêt du dispositif,
- un système double/bouton 88 pour déclencher et sécuriser le tir : le système de double bouton (deux boutons sur les deux faces opposées) joue un rôle de sécurisation du tir dans le sens où le tir n'est possible que lorsque les deux boutons sont maintenus appuyés par l'utilisateur. Dès que l'utilisateur relâche un seul des deux boutons, le tir est immédiatement stoppé. Par voie de conséquence, si malencontreusement la pièce à main était posée sur un plan de travail et qu'un des deux boutons venait en appui sur un élément du plan de travail, le tir ne peut pas se déclencher.
- un voyant lumineux 89 indiquant à l'utilisateur que le dispositif en cours de fonctionnement,
- un buzzer 717 indiquant un problème à l'utilisateur (problème de batterie, température mesurée par le pyromètre trop élevée).

Avantageusement, il est prévu selon un mode de réalisation possible de l'invention un manchon stérile amovible 80 destiné à contenir le dispositif et l'isoler de l'environnement extérieur. Ce manchon peut être interprété comme une enveloppe stérile dans lequel le dispositif est placé afin de garantir la stérilité du lieu d'utilisation du dispositif. Avantageusement, le manchon amovible 80 est conçu de façon à ne pas interférer avec le faisceau laser. A cet effet, il est prévu une fenêtre transparente aux longueurs d'onde prédéfinies du dispositif apte à laisser passer un faisceau dont la longueur d'onde est choisie parmi les longueurs d'onde prédéfinies. Le manchon amovible 80 est également conçu de façon à ne pas interférer avec le fonctionnement du pyromètre 712. Le manchon amovible 80 permet également l'évacuation de la chaleur sans avoir d'effet néfaste pour l'environnement stérile dans lequel le dispositif de traitement 70 est utilisé. Avec ce manchon il est possible de réaliser le tir laser sans rompre la stérilité de la face extérieure du manchon.

Le manchon doit évidemment être étanche et être muni d'un système de fermeture étanche également, afin de constituer une barrière microbienne entre le dispositif et le milieu extérieur. Cette barrière microbienne ne doit pas pour autant gêner l'évacuation de la chaleur engendrée par le dispositif. Le manchon contient donc au moins un filtre à air et de préférence au moins deux filtres à air (une entrée d'air et une sortie d'air) qui permettront la circulation de l'air dans le manchon.

Le manchon ne doit également pas gêner l'accès aux commandes du dispositif (boutons de tir, interrupteur, bouton d'arrêt d'urgence), la lecture des informations (écran LCD, voyants lumineux), ni la préhension du dispositif. Dans un mode de réalisation privilégié, le manchon sera donc constitué d'une partie rigide en bas du dispositif de façon à pouvoir le positionner correctement, et transparente et souple sur la partie recouvrant le reste du dispositif.

Le manchon étant en contact avec le patient au niveau des surfaces 81 et 82, et notamment dans le cas de traitement d'une incision, le risque que le manchon soit en contact avec du sang ne doit pas être négligé. En effet, si le manchon est en contact avec du sang, le sang peut s'accumuler sur celui-ci lors du déplacement du manchon sur le reste de l'incision ; le faisceau laser traversant le manchon pourrait alors être absorbé par ces traces, et perdre en conséquence une partie de l'énergie de traitement dans cette zone. Dans un mode de réalisation privilégié, le dispositif de traitement 70 et la partie basse et rigide 87 du manchon 80 en contact avec l'endroit à traiter comportent tout deux, un décroché 83 en face de la sortie du faisceau laser, qui permettra de ne pas être en contact direct avec le patient et donc de ne pas être salit ou contaminé et de garantir une distance entre la sortie du moyen de mise en forme du faisceau et la zone de tissu à traiter.

Ainsi, le manchon comprend une partie rigide et une partie flexible (pour la manipulation des boutons et sa prise en main). Il est prévu que les deux parties soient en PVC, les épaisseurs sont par exemple comprises entre 2 et 3 dixièmes de millimètres, pour la partie flexible et de quelques millimètres pour la partie rigide.

### Support RFID

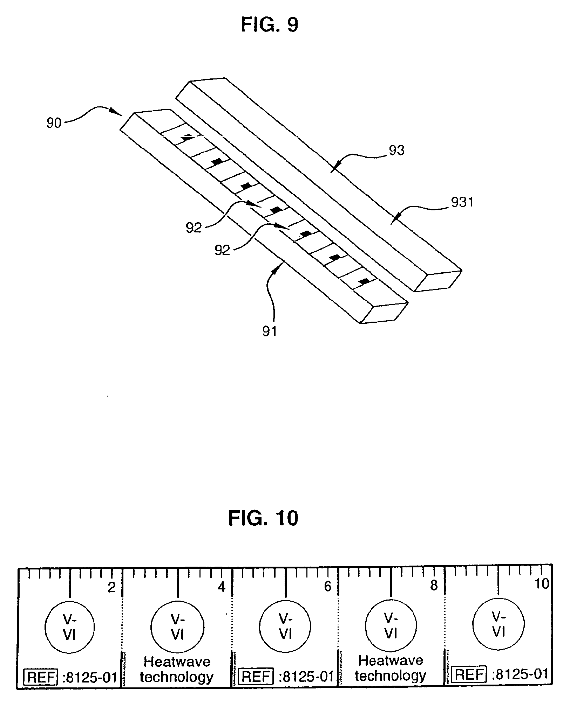

En référence à la figure 9, et comme décrit précédemment, il est prévu de sécuriser et paramétrer le traitement grâce à une bandelette de sécurité 90 contenant une puce RFID qui communiquera au dispositif laser les différents paramètres de fonctionnement en fonction de l'indication d'utilisation et du phototype du patient à traiter. Cette bandelette de sécurité 90, qui peut être déclinée en plusieurs longueurs (4, 10 et 20 cm par exemple), est collée à environ 5 mm de la zone à traiter.

La bandelette de sécurité est composée de deux supports adhésifs : un support adhésif 91 en double face et l'autre support adhésif 93 en simple face. Ces deux supports prennent en sandwich les supports RFID 92 qui seront positionnées tous les 2 cm. La bandelette de sécurité peut avoir par exemple une largeur de 2 cm.

Le support adhésif inférieur 91 est en contact avec la peau du patient. De ce fait, il doit répondre aux problématiques de biocompatibilité et de maintien suffisant sur la peau durant son utilisation. Le support adhésif inférieur 91 doit pouvoir coller sur la peau du patient et être assemblé aussi bien avec le support RFID qu'avec le support adhésif supérieur 93. De ce fait, le support adhésif inférieur est à double face adhésive.

Il est prévu, lors de la fabrication des bandelettes de sécurité, une étape de stérilisation.

Le support adhésif supérieur 93 doit être une adhésive simple face afin de permettre l'assemblage avec les supports RFID 92 et le support adhésif inférieur 91. En référence à la figure 10 et sur sa partie supérieure 93, il est prévu d'imprimer des informations telles que le phototype, le nom de l'entreprise, une graduation qui permettra au praticien de savoir où il en est par rapport au traitement ainsi que la référence du produit.

Le support adhésif supérieur 93 est tel qu'il n'altère pas la communication RFID entre le lecteur 711 (voir figure 7) du dispositif et les supports RFID 92.

Les supports RFID 92 doivent être conformes à la norme ISO 15693 -3. Il est d'ailleurs prévu d'implémenter les parties obligatoires et optionnelles telles que définies par cette présente norme. La distance de lecture du support RFID étant une donnée critique, les supports RFID 92 selon l'invention sont tels qu'ils permettent de fixer cette valeur une fois le couple « support RFID - Lecteur RFID » est choisi. Les supports RFID peuvent avoir par exemple une taille de 14 mm x 14 mm pour une épaisseur inférieure à 1 mm. Il est prévu que la capacité de stockage de l'EEPROM du support RFID soit au minimum de 1024 octets. De plus, un processus de stérilisation à l'oxyde d'éthylène est prévu.

### Procédé de communication entre le support RFID et le dispositif

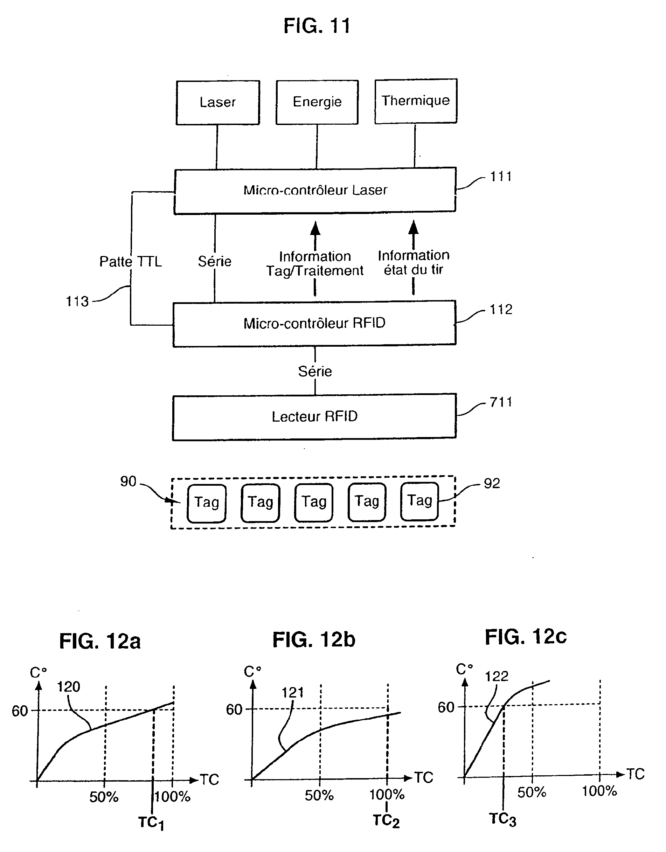

En référence à la figure 11, le dispositif est composé de deux microcontrôleurs 111 et 112. L'un (microcontrôleur 111) est responsable de la gestion du laser, de l'énergie, du contrôle thermique et de l'IHM ; l'autre (microcontrôleur 112) de la gestion des bandelettes de sécurités 90. La responsabilité de la gestion des tirs est, quant à elle, partagée par les deux microcontrôleurs 111 et 112. Le tir peut être stoppé à cause d'une température trop élevée (gérée par le microcontrôleur 111) ou bien d'une demande d'arrêt d'urgence (microcontrôleur 111). La reprise du traitement est contrainte par ces conditions d'arrêt.

En référence aux figures 12a, 12b et 12c, le pyromètre 712 (voir figure 7) arrête le tir si la température de la peau est supérieure à une température critique, 60°C par exemple (gestion par le microcontrôleur 111). Ces figures sont des graphes présentant des courbes 120, 121 et 122 de température (degré Celsius en ordonnées) en fonction du temps de chauffe TC en abscisse ; TC1, TC2 et TC3 représentent, quant à eux, les temps auxquels est arrêté le tir. Si le tir a été arrêté par le pyromètre 712, le tir peut à nouveau être autorisé, une seule fois, au bout d'un temps de sécurité (par exemple 5 secondes) si durant le premier tir, le temps de tir est inférieur ou égal à la moitié de la durée du tir donnée en paramètre (géré par le microcontrôleur 112). C'est le cas de la figure 12c. Ainsi les figures présentent les cas suivants :
- la figure 12a présente le cas où le tir est arrêté (TC1) au-delà de 50% de la durée du tir en paramètre ; dans ce cas le tir ne peut être repris,
- la figure 12b présente le cas où le tir n'est pas arrêté jusqu'au bout de la durée du tir en paramètre (TC2),
- la figure 12c présente le cas où le tir est arrêté (TC3) avant 50% de la durée de tir en paramètre ; dans ce cas le tir peut être repris.

Si le pyromètre 712 se déclenche au-delà de 50% (figure 12a) de la durée normale du tir, un bip doit signaler une fin de tir normal (microcontrôleur 111). Si le pyromètre 712 se déclenche en deçà de 50%, il faut un élément de l'IHM pour montrer à l'utilisateur qu'il peut récupérer le tir.

Si jamais l'utilisateur décroche (arrêt, volontaire ou non, dû à l'utilisateur) et que le temps d'arrêt est inférieur ou égal à 75% de la durée du tir en paramètre, une reprise du tir peut être autorisée une seule fois au bout de 5 secondes et ce, durant 60 minutes.

En cas de déclenchement de l'arrêt d'urgence (fig. 7, bouton 76), il peut être prévu une procédure de vérification à suivre.

La présence d'un support RFID 92 est indiquée par la patte TTL 113 du microcontrôleur 112. La présence d'un support RFID 92 ne donne pas d'information concernant son état. Pour connaître l'état du support RFID 92, il faut envoyer un message de lecture des paramètres au microcontrôleur 112. Cette commande permet de connaître l'état du traitement du support RFID 92 présent dans le champ du lecteur RFID 711. Les différents états d'un support RFID 92 sont :
- Traité ;
- OK (Non Traité) ;
- Reprise immédiate (identique que non traité) ;
- Attente avant reprise (5 secondes).

Le microcontrôleur 111 reconnaît cinq messages en provenance du microcontrôleur 112 :
- INIT : Initialisation de l'horloge et de l'identifiant du dispositif.
- PARAM : Récupérer l'état du support RFID 92, la puissance et la durée du tir (ne peut tirer que si l'état est OK).
- START Début de tir.
- STOP Fin du tir.
- PAUSE Arrêt prématuré du tir.

Au démarrage du dispositif, le microcontrôleur 111 initialise l'horloge du microcontrôleur 112 avec le message INIT. Les deux horloges se trouvent synchronisées. Au front montant de la patte TTL 113 du microcontrôleur 112, le microcontrôleur 111 demande les paramètres du tir avec le message PARAM. En fonction de la réponse du microcontrôleur 112, l'IHM est mise à jour et le tir autorisé. Pendant le tir, le microcontrôleur 111 indique l'avancement du tir au microcontrôleur 112 avec les messages START, STOP et PAUSE. Le microcontrôleur 112 met à jour les informations des supports RFID 92 en fonction de ces messages. L'horloge du dispositif est gérée par le microcontrôleur 111. Elle ne contient pas la date et l'heure exacte, mais une information de datation relative au démarrage du dispositif. Elle a une fonction de chronomètre. L'horloge du dispositif peut être réinitialisée suite à une interruption d'alimentation prolongée de la batterie. Dans ce cas, si un support RFID 92 a été traité, sa date de début de traitement est postérieure à la date courante. Même si le traitement n'est pas terminé, le support RFID 92 est considéré comme traité.

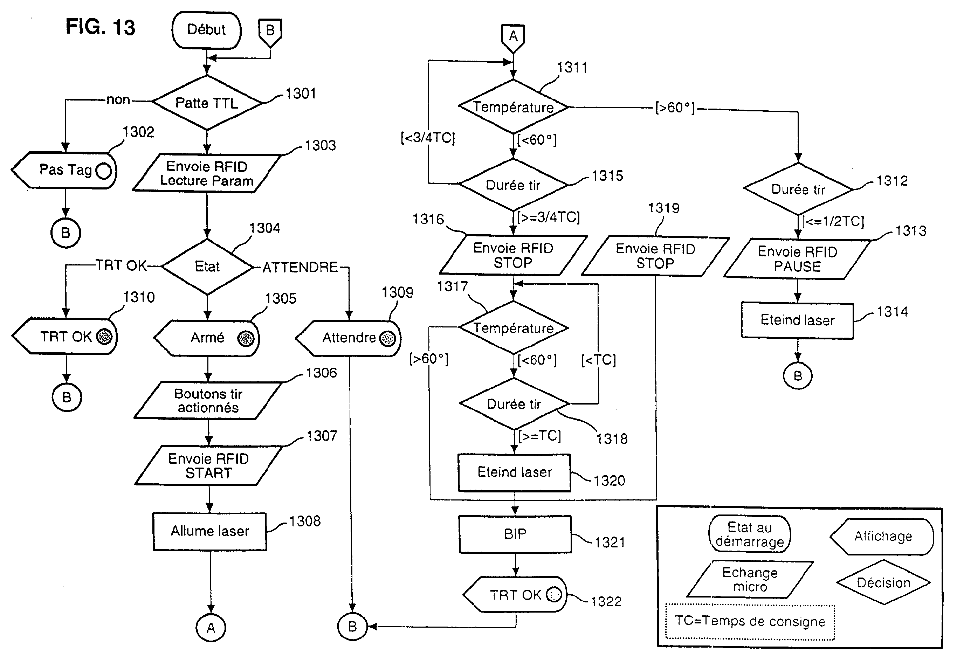

Sur la figure 13 est représenté un diagramme fonctionnel du microcontrôleur 111. Ainsi au bloc 1301 est détecté la présence d'un support RFID ou non ; dans ce dernier cas l'IHM indique à l'utilisateur qu'aucun support RFID n'est présent (boc 1302). Si un support RFID est détecté, une lecture des paramètres est initiée (bloc 1303) pour un diagnostique de l'état du support RFID (bloc 1304). Si l'état est OK alors le dispositif indique via son IHM qu'il est « armé » (bloc 1305) et si le bouton de tir est actionné (bloc 1306), alors le microcontrôleur 111 envoie au microcontrôleur 112 le message START 1307 indiquant le début d'un tir. Enfin le laser est allumé (bloc 1308). Si le message est ATTENDRE, alors le dispositif indique qu'il est « en attente » (bloc 1309) et revient au début du diagramme. Si le message est TRT OK (traité) alors le dispositif indique que la zone a été traitée (bloc 1310) et revient au début du diagramme.

Si un tir est initié (bloc A), une mesure 1311 de la température est effectuée. Si cette température est supérieure à 60°C, alors une mesure du temps 1312 est également effectuée. Si ce temps est inférieur à 50% de la durée du tir en paramètre, alors un message PAUSE 1313 est envoyée au microcontrôleur 112, et le laser est éteint (bloc 1314).

Si la température mesurée en 1311 est inférieur à 60°C, alors une mesure du temps 1315 est également effectuée. Si ce temps est inférieur à 75% de la durée du tir en paramètre, alors on recommence une mesure de la température 1311. Si, par contre, le temps est supérieur à 75% de la durée du tir en paramètre TC, alors un message STOP 1316 est envoyé au microcontrôleur 112. Une mesure de la température est alors effectuée (bloc 1317), si la température est inférieure à 60°C, alors on effectue une vérification sur la durée de tir (bloc 1318) ; si la durée du tir est inférieure à la durée du tir en paramètre TC (signifiant que le tir n'est pas encore terminé), alors on re-effectue une vérification sur la température (retour sur le bloc 1317). Cette boucle continue jusqu'à ce que la température dépasse 60°C ou jusqu'à ce que la durée de tir atteigne ou dépasse TC. Dans les deux cas, on éteint le laser (bloc 1320), on envoie un bip 1321 (ou tout autre dispositif IHM) afin de prévenir l'utilisateur de la fin du traitement de cette zone et on affiche un message de fin de traitement (bloc 1322). De retour au bloc 1312, si la durée de tir est supérieure à 50% de TC, alors un message STOP est envoyé au microcontrôleur 1319, puis on éteint le laser (bloc 1320). ). Si la durée du tir est inférieure ou égale à 50% de TC alors un message STOP est envoyé au microcontrôleur 1319 puis le laser est éteint.

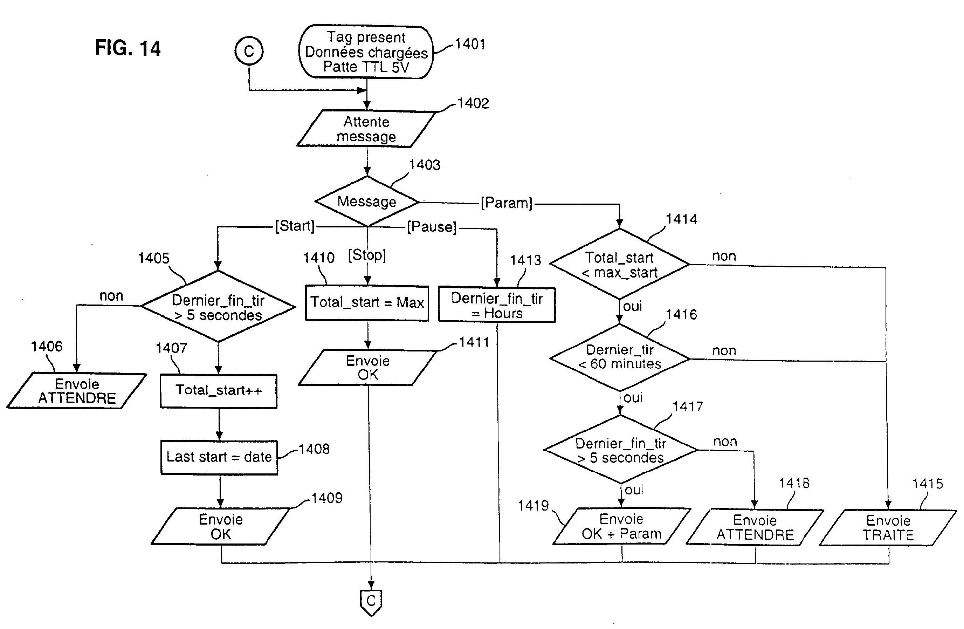

En référence à la figure 14, nous allons décrire un diagramme fonctionnel de gestion de l'autorisation de tir du microcontrôleur 112. Si un support RFID est détecté (bloc 1401), le microcontrôleur 111 se met en attente d'un message (bloc 1402). Lorsqu'un message (bloc 1403) est reçu, il est analysé. Si le message est START, et que le dernier tir date d'il y a moins de 5 secondes (1405), alors le microcontrôleur 111 envoie le message ATTENDRE (bloc 1406). Si, par contre, le dernier tir date d'il y a plus de 5 secondes, une variable total_start (indiquant le nombre de tir effectué) est incrémentée d'une unité (bloc 1407) et une variable last_start (indiquant la date du dernier tir) est mise à jour (bloc 1408). Enfin un message OK est envoyé (bloc 1409).

Si le message reçu est STOP, alors la variable total_start est fixée à un maximum prédéterminé (bloc 1410) et le message OK est envoyé (bloc 1411).

Si le message reçu est PAUSE, on enregistre la date de fin de tir (bloc 1413).

Enfin, si le message reçu est PARAM, on analyse la variable total_start (bloc 1414). Si cette dernière est inférieur au maximum prédéfini, alors le message TRAITE est envoyé (1415). Dans le cas contraire, on analyse la variable last_start (bloc 1416), si ce dernier est inférieur supérieur à 60 minutes, alors le message TRAITE est envoyé (bloc 1415). Dans le cas contraire on vérifie que le dernier tir date d'il y a plus de 5 secondes (bloc 1417); si ce n'est pas le cas, on envoie le message ATTENDRE (bloc 1418). Si, par contre, le dernier tir date d'il y a plus de 5 secondes, le message OK est envoyé et une lecture des paramètres est effectué (bloc 1419). Les informations contenues dans le support RFID peuvent être choisies parmi les suivantes : identifiant unique du support RFID, date de fabrication, date de péremption, identifiant de paramétrage du dispositif en fonction du type de peau, numéro de lot de fabrication de la bandelette de sécurité.

Bien évidement, l'ensemble des valeurs numériques (pourcentages, température, ...) de la description ne sont données qu'à titre purement indicatif afin d'orienter la réalisation de l'invention. On pourra donc utiliser d'autres valeurs numériques par exemple déterminés par expérimentation sans sortir du cadre de l'invention.

Selon une réalisation possible de l'invention, il est prévu que le support adhésif ait un triple rôle sécuritaire :
- la sécurisation du dispositif, dans le sens ou le fonctionnement du dispositif est immédiatement bloqué dès que le support RFID 92 inclus dans le support adhésif 90 sort du champ du lecteur RFID 711 compris dans le dispositif.
- la définition des paramètres de traitement. L'utilisateur choisi le support adhésif en fonction de l'indication et du type de peau du patient, et les supports RFID 92 préprogrammés dans le support adhésif 90 envoient directement les paramètres adéquats au dispositif.

- la protection de son intégrité (usage unique). Lors de son utilisation, le dispositif inscrit les informations relatives à l'état du traitement sur les supports RFID 92 contenus dans le support adhésif. Les états de traitement sont : la zone à traiter n'a pas été traitée, la zone à traiter est en cours de traitement, et le traitement de la zone à traiter est terminé. Dès lors que le traitement est terminé, le dispositif recevant l'information relative à l'état de la zone à traiter comporte des moyens d'empêcher une seconde utilisation du support adhésif 90 muni d'un moyen d'identification lorsque celui-ci a déjà été utilisé une première fois pour traiter une zone à traiter. Ce principe de verrouillage des supports RFID permet donc de s'assurer que chaque support adhésif ne sera utilisé qu'une seule fois. L'avantage direct de ce système est l'impossibilité :
   o d'appliquer un traitement deux fois au même endroit (élimination du risque de surdosage)
   o de réutiliser un support adhésif livré stérile qui ait déjà été utilisé

Il est prévu que les moyens d'empêcher une seconde utilisation du support adhésif 90 soient directement compris dans le support RFID lui-même.

### Cas des surfaces à traiter

Dans certaines applications médicales ou dermatologiques, le traitement doit être appliqué sur des surfaces et non sur des zones « linéaires » comme c'est le cas par exemple pour une incision.

Le dispositif doit donc être déplacé sur l'ensemble de la surface à traiter, ce qui rend difficile ou impossible l'apposition d'une bandelette de sécurité comme décrite précédemment le long de la zone à traiter. En effet, lorsque la zone à traiter est « linéaire », une telle bandelette peut être facilement posée à coté et le long de cette zone à traiter. Ce n'est pas le cas lorsque la zone à traiter est une surface. Il existe également d'autres raisons pour lesquelles une telle bandelette de sécurité ne peut être posée à coté de la zone à traiter : par exemple selon la position de la zone à traiter sur le corps l'apposition d'une telle bandelette peut être délicate (articulations, endroits) ou selon la nature de la peau de la zone à traiter, l'apposition d'une telle bandelette peut poser certains problèmes (allergie, apposition sur une muqueuse, ...etc.).

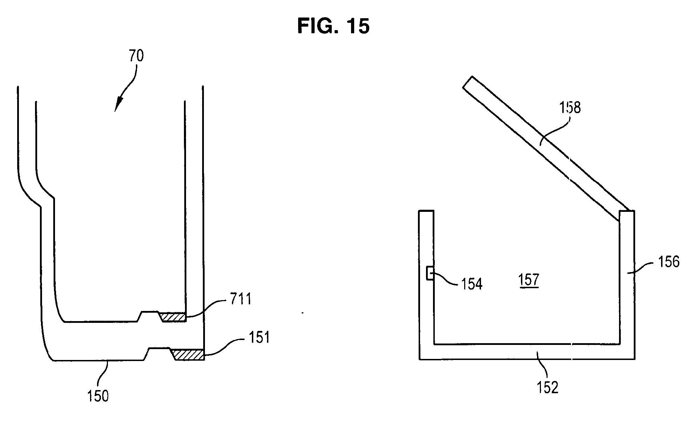

En référence à la figure 15, et afin de résoudre les éventuelle difficultés liées à l'apposition d'un manchon de stérilisation, il est prévu de munir le manchon de stérilisation 150 (identique au manchon de stérilisation 80) d'un support RFID 151 tel que celui décrit au sein d'une bandelette de sécurité, c'est-à-dire que le manchon de stérilisation 150 comprend un support RFID 151, lui-même comprenant des espaces de stockage destinés notamment à enregistrer une information relative aux paramètres de tir et/ou aux autorisations de tir comme décrit précédemment.

Le praticien choisit donc le manchon en fonction du patient à traiter et/ou du type de traitement à effectuer. Lorsque le manchon est correctement positionné sur le dispositif de traitement, le lecteur RFID du dispositif lit les informations et les paramètres de tir contenus dans le support RFID du manchon. Ainsi le dispositif fonctionne en combinaison avec le manchon (et plus spécifiquement avec le support RFID du manchon). Cet ensemble de traitement manchon/dispositif de traitement est amélioré par l'interaction entre le support RFID du manchon et lecteur RFID du dispositif. Une telle combinaison manchon/dispositif de traitement permet d'assurer que le dispositif est bien utilisé avec un manchon et donc d'assurer la stérilisation lors du traitement : en effet si le dispositif est utilisé sans le manchon, il ne reçoit aucun paramètre de tir, ni aucune autorisation de tir.

Les paramètres de fonctionnement de la source d'énergie sont mémorisés dans le support RFID du manchon et sont communiqués au dispositif pour la commande de la source d'énergie (par exemple le laser). Préférentiellement le support RFID fonctionne dans la bande comprise entre 13 et 14 MHz.

Cependant, dans le cas où la zone à traiter est une surface, le traitement est généralement plus long que dans le cas d'une zone « linéaire ». Ainsi il est prévu, selon une réalisation particulière de l'invention que le support RFID du manchon n'empêche pas une seconde utilisation et autorise autant de tirs que nécessaire pour le traitement de la surface.

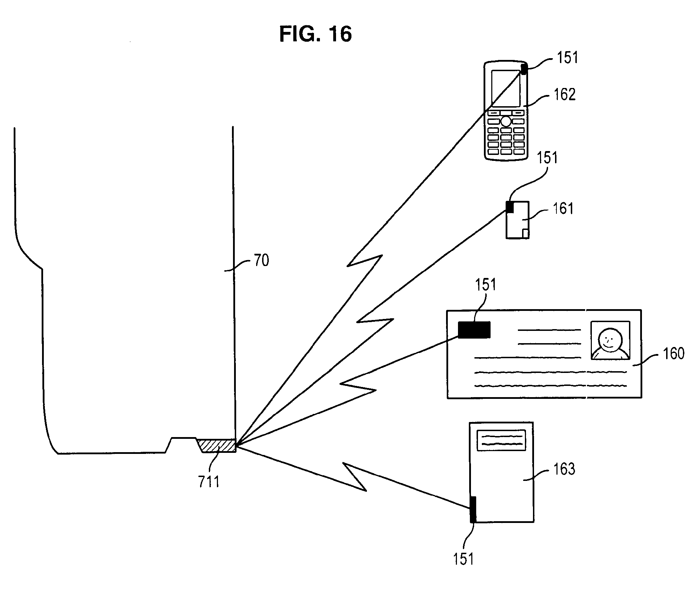

En référence à la figure 16, il est également prévu que le support RFID 151 soit compris dans un autre élément tel qu'une carte d'identification du praticien 160 ou tout autre élément portatif (badge 161, téléphone 162, bipper 163, ...).

Ainsi un praticien voulant utiliser le dispositif 70 doit se munir de cet élément portatif 160, 161, 162 ou 163 comprenant le support RFID 151 afin de pouvoir utiliser le dispositif de traitement 70. De cette manière, le lecteur 711 du dispositif 70, en communication avec le support RFID 151 de l'élément portatif peut lire les informations relatives aux paramètres de fonctionnement et/ou aux autorisations de tir. Une telle réalisation de l'invention permet que seul le porteur de cet élément portatif comprenant le support RFID puisse utiliser le dispositif de traitement 70.

### Stérilisation du manchon

Le manchon étant l'équipement en contact avec la peau du patient et plus généralement avec l'environnement du patient, un procédé de stérilisation du manchon est essentiel et indispensable de façon à ce que le manchon ne puisse pas être utilisé sans qu'une stérilisation préalable ait été effectuée.

En référence à la figure 15, il est prévu que le support RFID 151 du manchon 150, comprenne des espaces de stockage destinés notamment à enregistrer une information relative à la stérilisation du manchon. Cette information est caractéristique d'un état de stérilisation du manchon. Il est prévu parallèlement qu'un stérilisateur 152 destiné à la stérilisation du manchon 150 comprenne un dispositif RFID 154 apte à lire et à écrire sur les espaces de stockage dudit support RFID 151 du manchon 150. On rappelle qu'un stérilisateur médical est un instrument de médecine qui permet de stériliser les outils et accessoires médicaux. Selon un principe de fonctionnement, le stérilisateur comprend un tube provoquant un échauffement au sein du stérilisateur. Ce tube émet des rayons ultra-violets ; ces derniers frappent les parois intérieures du stérilisateur. Les ultra-violets transforment l'oxygène de l'air ambiant en ozone et stérilisent les outils ; ces rayons ont une puissance germicide élevée. Le fonctionnement du stérilisateur est notamment garantit par une porte 158 (combinée à des parois 156) apte est fermer hermétiquement l'enceinte 157 du stérilisateur 152.

Un procédé de stérilisation du manchon 150 comprend donc les étapes suivantes :
- placer le manchon 150 dans l'enceinte 157 du stérilisateur 152,
- refermer la porte hermétique 158,
- lire les informations contenues dans le support RFID 151 du manchon 150 permettant de savoir si oui ou non le manchon est stérile (auquel cas il est prévu d'avertir l'utilisateur du stérilisateur que le manchon a déjà été stérilisé ; il peut être prévu de démarrer le procédé de stérilisation),
- lancer un procédé de stérilisation,
- à la fin du procédé de stérilisation, modifier à l'aide du dispositif RFID 154, l'information inscrite sur l'espace de stockage selon laquelle le manchon a bien été stérilisé ainsi que la date de la stérilisation,

Un procédé d'utilisation du manchon 150 comprend donc les étapes suivantes :
- placer le dispositif de traitement selon l'invention au sein du manchon de stérilisation,
- le lecteur RFID 711 du dispositif selon l'invention interroge le support RFID du manchon afin de savoir si oui ou non le manchon a été stérilisé et la date de sa dernière stérilisation.
- décider à partir de ces informations si le manchon peut ou pas être utilisé. La question relative est de savoir si oui ou non le manchon peut être considéré comme stérile ; une autorisation de tir pour le dispositif dépend de cette décision.
- Si le manchon ne doit pas être utilisé (manchon non stérile), activer une alarme visuel et/ou sonore afin d'avertir l'utilisateur que le manchon ne doit pas être utilisé. Il est également prévu que le dispositif n'autorise aucun tir dans ce cas. Si le manchon est considéré comme stérile, alors un tir du dispositif est autorisé.

La décision selon laquelle le manchon est stérile ou non n'est prise à partir des informations recueillies sur la puce RFID 151 du manchon 150. Si ces informations indique que le manchon n'est pas stérile ou que la dernière stérilisation du manchon date de plus d'un certain temps défini (par exemple 24 heures), alors la stérilisation n'est plus garantie et la décision est prise que le dispositif de traitement peut être utilisé.

Par contre, si d'après ces informations, le manchon a bien été stérilisé et que la stérilisation date de moins du temps déterminé, alors la décision est prise selon laquelle le dispositif est stérile et qu'il peut être utilisé.

Après une utilisation du couple manchon/dispositif de traitement tel que décrit précédemment, il est prévu que le lecteur RFID 711 du dispositif de traitement modifie les informations contenue dans le support RFID 151 du manchon de stérilisation 150 indiquant que le manchon a été utilisé et qu'il n'est donc plus stérile. Ainsi si le manchon est utilisé ultérieurement sans une stérilisation préalable, un avertissement sera porté à l'utilisateur selon lequel le manchon n'est pas stérile et un tir n'est pas autorisé.

*Etude clinique en double aveugle* / *Etude randomisée.*

Dans un certain cadre d'utilisation, l'ensemble lecteur RFID et bandelette de sécurité servent également à réaliser une étude clinique dit en « double aveugle ». En plus de contenir les paramètres du laser, la bandelette contient une information stipulant si le laser doit effectuer un tir réel ou simplement le simuler. Il est prévu que le dispositif de traitement, lorsque ce dernier traite réellement ou pas la zone à traiter ait le même comportement perceptible par le praticien (émission d'un son ou d'un signal visuel indiquant le tir). La programmation des bandelettes est effectuée lors de leur fabrication par un logiciel qui aléatoirement autorise ou non le traitement « réel » à travers les paramètres contenu dans le support RFID de la bandelette.

Ce même logiciel permet de lire les informations concernant l'autorisation du traitement. Le praticien traite alors le patient à l'aide de la bandelette sans savoir si le traitement est réellement délivré par le dispositif de traitement. Après l'utilisation de la bandelette, le praticien remet la bandelette à une personne chargée de l'étude clinique avec l'appréciation qu'il fait du résultat du traitement. La personne chargée de l'étude lit alors la bandelette afin de déterminer si le patient a été traité ou non.

Ainsi, avec un tel procédé, aucun intervenant direct ne peut savoir quand le dispositif effectue le traitement réellement ou non durant l'essai clinique. Ce principe permet de recueillir des avis sur le résultat du traitement obtenus d'une complète objectivité.

## Revendications

1. Dispositif de traitement dermatologique comprenant une source d'énergie, délivrant un faisceau laser de profil de répartition spatiale de puissance quasi-gaussien, et un comprenant des moyens de mise en forme du faisceau,
***caraciérisé en ce qu'il*** comprend des moyens de mise en forme du faisceau, entre la source d'énergie et une zone à traiter, adaptés pour convertir le faisceau en un faisceau de profil de répartition spatiale de puissance quasi-linéaire, présentant un taux de variation inférieur à 5% le long de la largeur de front, tout en gardant un rapport entre largeur de front/largeur à mi-hauteur supérieur à 90% , afin d'obtenir un chauffage homogène sur la zone à traiter, le dispositif de traitement dermatologique étant **caractérisé en ce que** les moyens de mise en forme du faisceau comprennent au moins un réseau de lentilles (64) et une lentille cylindrique (66).

2. Dispositif de traitement selon l'une des revendications précédentes, **caractérisé en ce qu'**il comprend une fibre optique (63) placée devant la source d'énergie (62) afin de diminuer la divergence de l'axe rapide de la source d'énergie (62).

3. Dispositif de traitement selon l'une des revendications précédentes, **caractérisé en ce qu'**il comprend un moyen de pyrométrie (712).

4. Dispositif de traitement selon l'une des revendications précédentes, **caractérisé en ce qu'**il comprend une interface d'utilisation comprenant au moins l'un des éléments suivants : un écran LCD (75), un bouton d'arrêt d'urgence (76), un bouton marche/arrêt (77), un système double/bouton (88), un voyant lumineux (89) et un buzzer (717).

5. Dispositif de traitement selon l'une des revendications précédentes, **caractérisé en ce que** la source d'énergie est une diode (62) de longueur d'onde comprise entre 800 et 1800nm, et préférentiellement entre 810nm et 910nm.

6. Dispositif de traitement selon l'une des revendications précédentes, **caractérisé en ce qu'**il comprend un moyen de batterie (71) afin de rendre le dispositif autonome.

7. Dispositif de traitement selon l'une des revendications précédentes, **caractérisé en ce qu'**il comprend un moyen de lecture RFID (711) afin de sécuriser l'utilisation du dispositif.

8. Dispositif de traitement selon la revendication précédente, **caractérisé en ce que** la portée du lecteur RFID est inférieure à 5 mm.

9. Dispositif de traitement dermatologique selon l'une des revendications précédentes, **caractérisé en ce qu'**il comprend un microcontrôleur (111) destiné à la gestion de la source d'énergie.

10. Dispositif de traitement dermatologique selon l'une des revendications précédentes, **caractérisé en ce qu'**il comprend un décroché (83) en aval de la sortie des moyens de mise en forme du faisceau laser afin de garantir une distance entre la sortie des moyens de mise en forme et une zone à traiter.

11. Ensemble de traitement comprenant un dispositif selon l'une des revendications 1 à 10, **caractérisé en ce qu'**il comprend en outre un manchon de stérilisation (80).

12. Ensemble de traitement dermatologique selon la revendication 11, **caractérisé en ce qu'**il comprend, un support RFID (151) compris dans le manchon de stérilisation (80).

13. Equipement de traitement dermatologique comprenant un dispositif selon l'une des revendications 1 à 10 et un support RFID **caractérisé en ce que** le support RFID (92) est destiné à transmettre au dispositif des informations concernant une zone à traiter.

14. Equipement de traitement dermatologique selon la revendication précédente **caractérisé en ce que** le dispositif comprend un microcontrôleur (112) destiné à la gestion de la lecture du support RFID (92).

15. Equipement de traitement dermatologique selon l'une des revendications 13 et 14, **caractérisé en ce que** le support RFID (92) interagit avec la source d'énergie grâce à un contact.

16. Equipement de traitement dermatologique selon l'une des revendications 13 à 14, **caractérisé en ce que** les paramètres de fonctionnement de la source d'énergie sont mémorisés dans le support RFID (92) et communiqués au dispositif pour la commande de la source d'énergie.

17. Equipement de traitement dermatologique selon l'une des revendications 13 à 16, **caractérisé en ce que** le support RFID (92) fonctionne dans la bande comprise entre 13 et 14 MHz.

18. Equipement de traitement dermatologique selon l'une des revendications 13 à 17, **caractérisé en ce que** le support RFID (92) comprend des moyens de stockage d'une information caractérisant un état d'une zone à traiter, l'information correspondant à l'un des états suivants :
- la zone à traiter n'a pas été traitée,
- la zone à traiter est en cours de traitement,
- le traitement de la zone à traiter est terminé.

19. Equipement de traitement dermatologique selon la revendication 13 à 18, **caractérisé en ce que** le dispositif, recevant l'information correspondant à l'état de la zone à traiter comporte des moyens d'empêcher une seconde utilisation du support adhésif muni d'un support RFID (92) lorsque celui-ci a déjà été utilisé une première fois pour traiter la zone à traiter.

20. Equipement de traitement dermatologique selon l'une des revendications 13 à 19, **caractérisé en ce que** le support RFID (92) est compris dans un support adhésif (90), le support adhésif (90) étant positionné à proximité d'une zone à traiter.

21. Equipement de traitement dermatologique selon la revendication 20, **caractérisé en ce que** le support RFID (92) comprend des moyens empêchant une seconde utilisation du support adhésif (90) si celui-ci a déjà été utilisé pour le traitement d'une zone à traiter.

22. Ensemble de traitement, **caractérisé en ce qu'**il comprend un manchon tel que défini dans l'une des revendications 11 ou 12 et un dispositif de traitement tel que défini dans l'une des revendications 1 à 10 en combinaison avec la revendication 7.

## Patentansprüche

1. Dermatologische Behandlungsvorrichtung, eine Energiequelle umfassend, die einen Laserstrahl mit einem fast-gaussförmigen Profil der räumlichen Leistungsverteilung aussendet, und einen abnehmbaren und austauschbaren Optikblock, **dadurch gekennzeichnet, dass** sie Strahlformungsmittel umfasst, die mindestens ein Linsensystem (64) und eine zylindrische Linse (66) zwischen der Energiequelle und einem zu behandelnden Bereich aufweist, dazu eingerichtet, den Strahl in einen Strahl mit einem fastrechteckigen Profil der räumlichen Leistungsverteilung umzuformen, der eine Abweichung von weniger als 5% über die Breite der Front aufweist und dabei ein Verhältnis zwischen Breite der Front und Breite in halber Höhe von mehr als 90% bewahrt, um eine homogene Erwärmung des zu behandelnden Bereichs zu erzielen.

2. Behandlungsvorrichtung nach einem der vorangehenden Patentansprüche, **dadurch gekennzeichnet, dass** sie eine Lichtleitfaser (63) vor der Energiequelle (62) angeordnet umfasst, um die Divergenz der schnellen Achse der Energiequelle (62) zu verringern.

3. Behandlungsvorrichtung nach einem der vorangehenden Patentansprüche, **dadurch gekennzeichnet, dass** sie ein Pyrometerorgan (712) umfasst.

4. Behandlungsvorrichtung nach einem der vorangehenden Patentansprüche, **dadurch gekennzeichnet, dass** sie eine Benutzerschnittstelle umfasst, die mindestens eins der folgenden Elemente aufweist: Flüssigkristallbildschirm (75), Notausschalter (76), Start-/Stoppschalter (77), Doppelschalter (88), eine Leuchtanzeige (89) und einen Summer (717).

5. Behandlungsvorrichtung nach einem der vorangehenden Patentansprüche, **dadurch gekennzeichnet, dass** die Energiequelle eine Diode (62) einer Wellenlänge zwischen 800 und 1800 nm ist, vorzugsweise zwischen 810 nm und 910 nm.

6. Behandlungsvorrichtung nach einem der vorangehenden Patentansprüche, **dadurch gekennzeichnet, dass** sie ein Batterieorgan (71) umfasst, um die Vorrichtung netzunabhängig zu machen.

7. Behandlungsvorrichtung nach einem der vorangehenden Patentansprüche, **dadurch gekennzeichnet, dass** sie ein RFID-Lesegerät (711) umfasst, um die Sicherheit der Verwendung der Vorrichtung sicherzustellen.

8. Behandlungsvorrichtung nach dem vorangehenden Patentanspruch, **dadurch gekennzeichnet, dass** die Reichweite des RFID-Lesers geringer ist, als 5 mm.

9. Dermatologische Behandlungsvorrichtung nach einem der vorangehenden Patentansprüche, **dadurch gekennzeichnet, dass** sie einen Mikrosteuerbaustein (111) umfasst, der der Steuerung der Energiequelle dient.

10. Dermatologische Behandlungsvorrichtung nach einem der vorangehenden Patentansprüche, **dadurch gekennzeichnet, dass** sie einen Rücksprung (83) hinter dem Austritt aus den Formungsmitteln umfasst, um einen Abstand zwischen den Formungsmitteln und dem zu behandelnden Bereich sicherzustellen.

11. Behandlungssatz, eine Vorrichtung nach einem der Patentansprüche 1 bis 10 umfassend, **dadurch gekennzeichnet, dass** er außerdem eine Sterilisierhülle (80) umfasst.

12. Dermatologischer Behandlungssatz nach Patentanspruch 11, **dadurch gekennzeichnet, dass** er ein RFID-Etikett (151) innerhalb der Sterilisierhülse umfasst.

13. Dermatologische Behandlungsausrüstung, eine Vorrichtung nach einem der Patentansprüche 1 bis 10 und ein RFID-Etikett umfassend, **dadurch gekennzeichnet, dass** das RFID-Etikett (92) dazu bestimmt ist, der Vorrichtung Informationen über einen zu behandelnden Bereich zu übermitteln.

14. Dermatologische Behandlungsausrüstung nach dem vorangehenden Patentanspruch, **dadurch gekennzeichnet, dass** die Vorrichtung einen Mikrosteuerbaustein (112) umfasst, der der Steuerung der Lesung des RFID-Etiketts (92) dient.

15. Dermatologische Behandlungsausrüstung nach einem der Patentansprüche 13 und 14, **dadurch gekennzeichnet, dass** das RFID-Etikett (92) über einen Kontakt mit der Energiequelle in Verbindung tritt.

16. Dermatologische Behandlungsausrüstung nach einem der Patentansprüche 13 bis 14, **dadurch gekennzeichnet, dass** die Betriebsparameter der Energiequelle im RFID-Etikett (92) gespeichert sind und der Vorrichtung zur Steuerung der Energiequelle mitgeteilt werden.

17. Dermatologische Behandlungsausrüstung nach einem der Patentansprüche 13 bis 16, **dadurch gekennzeichnet, dass** das RFID-Etikett (92) im Frequenzbereich zwischen 13 und 14 MHz arbeitet.

18. Dermatologische Behandlungsausrüstung nach einem der Patentansprüche 13 bis 17, **dadurch gekennzeichnet, dass** das RFID-Etikett (92) Speichermittel für eine Information aufweist, die den Zustand eines zu behandelnden Bereichs charakterisiert, wobei die Information einem der folgenden Zustände entspricht:
- der zu behandelnde Bereich ist nicht behandelt worden,
- der zu behandelnde Bereich ist in Behandlung,
- die Behandlung des zu behandelnden Bereichs ist abgeschlossen.

19. Dermatologische Behandlungsausrüstung nach einem der Patentansprüche 13 bis 18, **dadurch gekennzeichnet, dass** die Vorrichtung, die die Information empfängt, die dem Zustand des zu behandelnden Bereichs entspricht, Mittel aufweist, um eine zweite Verwendung des Klebeträgers, der ein RFID-Etikett (92) trägt, zu verhindern, wenn dieser bereits ein erstes Mal verwendet wurde, um den zu behandelnden Bereich zu behandeln.

20. Dermatologische Behandlungsausrüstung nach einem der Patentansprüche 13 bis 19, **dadurch gekennzeichnet, dass** das RFID-Etikett (92) sich in einem Klebeträger (90) befindet, wobei der Klebeträger (90) nahe einem zu behandelnden Bereich angeordnet ist.

21. Dermatologische Behandlungsausrüstung nach Patentanspruch 20, **dadurch gekennzeichnet, dass** das RFID-Etikett (92) Mittel aufweist, die eine zweite Verwendung des Klebeträgers (90) verhindern, wenn dieser bereits für die Behandlung eines zu behandelnden Bereiches verwendet wurde.

22. Behandlungssatz, **dadurch gekennzeichnet, dass** er eine Hülle umfasst, wie sie in einem der Patentansprüche 11 oder 12 definiert wurde, und eine Behandlungsvorrichtung, wie sie in einem der Patentansprüche 1 bis 10 definiert wurde, kombiniert mit dem Patentanspruch 7.

## Claims

1. Dermatological treatment device comprising an energy source emitting a quasi-Gaussian spatial power distribution profile laser beam, and a detachable and interchangeable optical unit, **characterized in that** it includes means of shaping the beam, comprising at least one lens array (64) and one cylindrical lens (66) between the energy source and the zone to be treated suitable for converting the beam into a beam with a flat-top spatial power distribution profile having a rate of variation of less than 5% along the wavefront width and a ratio of wavefront width to full-width half-maximum of greater than 90% for obtaining a homogeneous heating on the area to be treated.

2. Treatment device according to one of the preceding claims, **characterized in that** it includes an optical fiber (63) placed in front of the energy source (62) in order to reduce the divergence of the energy source (62) fast axis.

3. Treatment device according to one of the preceding claims, **characterized in that** it includes a means of pyrometry (712).

4. Treatment device according to one of the preceding claims, **characterized in that** it includes a user interface including at least one of the following components: an LCD screen (75), an emergency stop button (76), a on/off button (77), a double button system (88), an indicator light (89) and a buzzer (717).

5. Treatment device according to one of the preceding claims, **characterized in that** the energy source is a diode (62) the wavelength of which is between 800nm and 1800nm, and preferably between 810nm and 910nm.

6. Treatment device according to one of the preceding claims, **characterized in that** it includes a battery system (71) for autonomous operation.

7. Treatment device according to one of the preceding claims, **characterized in that** it includes an RFID reader (711) in order to ensure the device can be safely used.

8. Treatment device according to the preceding claim, **characterized in that** the range of the RFID reader is less than 5 mm.

9. Dermatological treatment device according to in one of the preceding claims, **characterized in that** it includes a microcontroller (111) for managing the energy source.

10. Dermatological treatment device according to one of the preceding claims, **characterized in that** it includes a recess (83) downstream of the laser beam shaper output in order to ensure there is a distance between the shaper output and the treatment zone.

11. Treatment kit comprising a device according to one of the preceding claims 1 to 10, **characterized in that** it further includes a sterilization sleeve (80).

12. Dermatological treatment device according to claim 11, **characterized in that** it includes an RFID component (151) included in the sterilization sleeve (80).

13. Dermatological treatment equipment including a device according to one of claims 1 to 10 and an RFID component, **characterized by** the fact that the RFID component (92) is designed to transmit information to the device about a treatment zone.

14. Dermatological treatment equipment according to the preceding claim, **characterized in that** the device includes a microcontroller (112) for managing reading of the RFID component (92).

15. Dermatological treatment equipment according to either of claims 13 and 14, **characterized in that** the RFID component (92) interacts with the energy source by means of a contact.

16. Dermatological treatment equipment according to any of claims 13 to 14, **characterized in that** the energy source operating settings are memorized in the RFID component (92) and communicated to the energy source control device.

17. Dermatological treatment equipment according to any of claims 13 to 16, **characterized in that** the RFID component (92) operates at a frequency of between 13 and 14 MHz.

18. Dermatological treatment equipment according to any of claims 13 to 17 **characterized in that** the RFID component (92) includes a means of storing information characterizing a status of an area to be treated, the information corresponding to one of the following statuses:
- the area to be treated has not been treated,
- the area to be treated is in the course of treatment,
- treatment of the area to be treated has finished.

19. Dermatological treatment equipment according to claim 13 to 18, **characterized in that** the device, upon receiving information about the status of the zone to be treated, includes means of preventing reuse of the adhesive attachment element containing an RFID component (92) when it has already been used once to treat the treatment zone.

20. Dermatological treatment equipment according to any of claims 13 to 19, **characterized in that** the RFID (92) component is contained in an adhesive element (90), the adhesive element (90) being positioned close to the area to be treated.

21. Dermatological treatment equipment according to claim 20, **characterized in that** the RFID component (92) includes means to prevent reuse of the adhesive attachment element (90) when it has already been used to treat a treatment zone.

22. Treatment unit, **characterized in that** it includes a sleeve as defined in one of the claims 11 or 12 and a treatment device as defined in one of the claims 1 to 10 in combination with claim 7.
